# EUROPEAN PATENT APPLICATION

(11) **EP 1 923 021 A2**
(43) Date of publication of application: **21.05.2008**
(21) Application number: 07254455.4
(22) Date of filing: 14.11.2007
(51) Int. Cl.: A61F 2/36

(54) **Short stem femoral prosthesis**

(30) Priority: 16.11.2006 GB 0622930
(71) Applicant: BENOIST GIRARD SAS, 14201 Hérouville-Saint-Clair Cédex (FR)
(72) Inventor: Mercuri, Mario, 40137 Bologna (IT); Falez, Francesco, 00189 Roma (IT); Vaienti, Francesco, 43100 Parma (IT); Massari, Leo, 44100 Ferrara (IT); Benazzo, Francesco, 27100 Pavia (IT); Masse, Alessandro, 10122 Torino (IT); Ruosi, Carlo, 80122 Napoli (IT); Marcucci, Massimiliano, 50134 Firenze (IT)
(74) Representative: Bridge-Butler, Alan James

(57) **Abstract**

A short stem femoral prosthesis comprising a proximal end portion having or adapted to receive a bearing ball head, and a gutter-shaped stem having anterior and posterior sides which extends from said proximal end portion to an open distal end curved in a medial/lateral plane through its length and closed by a substantially radially extending proximal end wall adjacent said proximal end portion.

## Description

This invention relates to a one-piece short femoral prosthesis.

Femoral prostheses are known which have a stem which, when fitted in a suitably prepared femur from which the head and at least part of the shoulder have been removed, extend downwards past the lesser trochanter and into the medullary canal in the shaft of the bone. Such stems can be fixed into the canal of the bone during implantation by utilising their tapered shape by cementing or other means. Prostheses of this type are well known.

Traditional neck filling stems of this type can require the removal of a large amount of trabeculae bone. Stress shielding can be expected and the consequent reabsorption of the neck can endanger the stability of the stem.

The present invention is intended to provide a construction which joins the concept of a proximal stem placed in the neck of the bone and following the anatomy of the boney trabeculae (bone sparing) and provide a design which aims to avoid the pitfalls shown up by former designs.

The stem of the present invention only extends for a short distance into the intramedullary canal and it is mainly located in a portion of the neck of the bone. A prosthesis employing this type of stem will therefore be referred to herein as a "short" femoral prosthesis, i.e. one which extends down the lesser trochanter but not into the diaphysis (shaft of the bone). Thus, its fixation is in the metaphysis. The length of the stem of such a "short" prosthesis for a medial size femur would be about 60 mm.

According to the present invention, a short stem femoral prosthesis comprises a proximal end portion having, or adapted to receive, a bearing ball head and a gutter-shaped stem having anterior and posterior sides which extends from said proximal end portion to an open distal end curved in a medial/lateral plane through its length and closed by a substantially radially extending proximal end wall adjacent said proximal end portion.

It is believed that a stem of this shape has the advantage to respect and spare the tractive trabecular system in the supero-lateral part of the femur. This portion is usually referred to as the principle tensile group and is usually not spared, event with conservative standard long stems.

The gutter shape allows the introduction of the stem with the minimal removal of bone, maximum rotational stability, full support from the bone, loose trabaculae will oppose the bending forces, large bone/metal interface, many invasive instruments which also help in terms of bone sparing.

The gutter along the profile of the stem can increase by 20% the surface area in contact with bone, thereby increasing the potential for boney in-growth onto the larger surface area.

In a preferred embodiment the gutter shaped stem has a medial side which is formed as a curve of constant radius from a first centre point and a lateral side which is also formed as a constant radius curve from a second centre point so that the stem tapers from its proximal end to its distal end.

In one preferred construction the gutter-shaped stem has an outer surface and an inner gutter surface which are both part-circular in cross-section.

In another construction the gutter-shaped stem can have an outer surface and an inner gutter surface which are both substantially square or rectangular in cross-section.

In yet another preferred embodiment the gutter-shaped stem can have an outer surface which is part-circular, square or rectangular and an inner gutter surface which has a semicircular lower portion and extended upper side walls in cross-section.

In any of the above constructions the gutter can be provided in the medial side of the stem or, alternatively, on the lateral side of the stem.

If desired the wall thickness of the gutter may be arranged to decrease proximally to distally.

The proximal end of the stem may be provided with a projecting flange or collar.

In a preferred embodiment the proximal end portion is provided with tapered boss to receive a co-operating bearing ball.

The prosthesis according to the invention may conveniently be made in one piece.

The invention also includes a short stem femoral prosthesis as set forth above in combination with a bearing ball.

The invention can be performed in various ways and some embodiments will now be described by way of example and with reference to the accompanying drawings in which :
Figure 1 is a side elevation of a one-piece "short" femoral prosthesis, according to the invention in which the gutter-shaped stem is of substantially square or rectangular cross-section and in which the gutter is provided on the lateral side of the stem;
Figure 2 is a cross-sectional side elevation of the prosthesis shown in Figure 1;
Figure 3 is an isometric view from above of the prosthesis shown in Figures 1 and 2;
Figure 4 is a cross-sectional side elevation of an alternative construction in which the cross-section of the gutter is substantially square or rectangular and the gutter is provided on the medial side of the stem;
Figure 5 is an end view of the gutter shown in Figure 4;
Figure 6 is an isometric view from above of the gutter shown in Figures 4 and 5;
Figure 7 is a side elevation of a prosthesis, according to the present invention, in which the gutter is of substantially circular cross-section and is on the lateral side of the stem;
Figure 8 is a cross-sectional view of the construction shown in Figure 7;
Figure 9 is an isometric view from above of the gutter show in Figures 7 and 8;
Figure 10 is a part isometric view from below of the prosthesis shown in Figures 7, 8 and 9;
Figure 11 is a side elevation of a prosthesis, according to the invention, in which the gutter is substantially part-circular and is located on the medial side of he stem;
Figure 12 is a cross-sectional side elevation of the prosthesis shown in Figure 11;
Figure 13 is an isometric view from above of the gutter shown in Figures 11 and 12;
Figure 14 is an isometric view from below of the prosthesis shown in Figures 11, 12 and 13;
Figure 15 is a plan view from above of a prosthesis according to the invention in which the outer surface of the stem is part-circular and the gutter surface has a semicircular lower portion and extended upper side walls in cross-section and also shows how a flange or collar can be provided;
Figure 16 is a cross-sectional side elevation of the prosthesis shown in Figure 15;
Figure 17 is an end view of the prosthesis shown in Figures 15 and 16;
Figure 18 is an isometric view from above of the construction shown in Figures 15, 16 and 17; and,
Figure 19 is an isometric view from below of the construction shown in Figures 15, 16, 17 and 18.

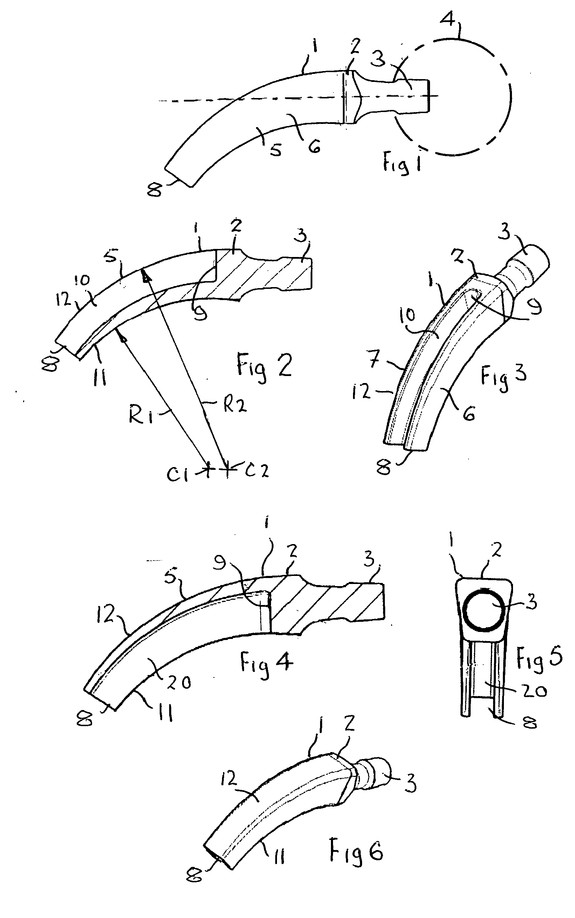

As shown in Figures 1, 2 and 3 a one-piece "short" femoral prosthesis 1 comprises a proximal end portion 2 which, in this construction, is provided with a tapered boss 3 to receive a co-operating bearing ball 4 shown in chain lines. In an alternative construction the bearing ball could be integral with the proximal end portion 2.

A gutter-shaped stem 5 is provided which has anterior and posterior sides 6 and 7, respectively, which extend from the proximal end portion 2 to an open distal end 8. The gutter shaped stem 5 is curved in a medial/lateral plane throughout its length and is closed by a substantially radially extending proximal end wall 9 adjacent the proximal end portion 2.

As will be seen, an open topped gutter 10 is provided in the stem 5 and in this construction is in the lateral side of the stem.

The gutter shaped stem 5 has a medial side 11 which is formed as a curve of constant radius R1 from a first centre point C1. The lateral side 12 is also formed as a constant radius, indicated by arrow R2, from a centre point C2 spaced away from the centre point C1 so that the stem 5 tapers from its proximal end at the proximal end portion 2 to its distal open end 8 as shown in Figure 2. The wall thickness of the gutter decreases proximally to distally.

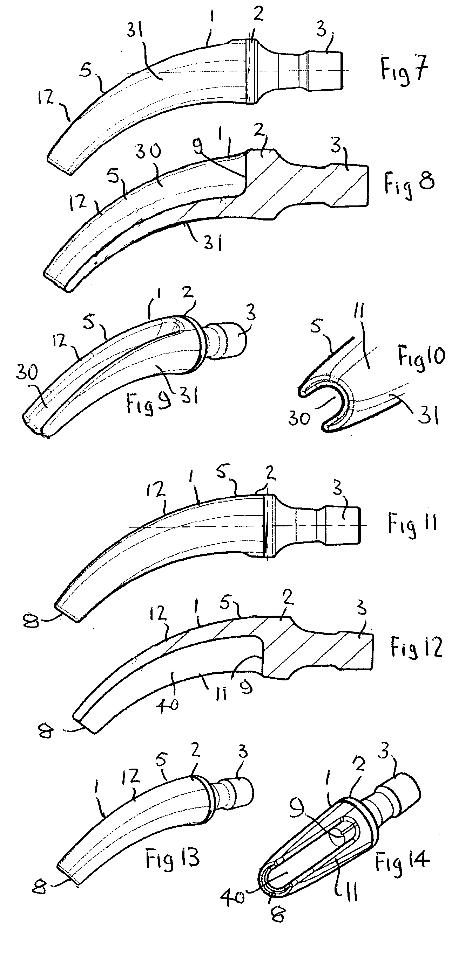

Figure 4 shows a construction similar to that shown in Figure 1, 2 and 3 and similar reference numerals are used to indicate similar parts. In this construction the gutter 20 is inverted to that shown in Figures 1, 2 and 3 and faces downwardly and is provided on the medial side 11 of the stem 5. The cross-sectional shape of the gutter is again substantially square or rectangular. Figures 7, 8, 9 and 10 show another construction in accordance with the invention in which the same reference numerals are used to indicate similar parts to those shown in Figures 1,2 and 3. The general construction is similar but the cross-section of the gutter 30 is part-circular, as is the outer surface 31. The gutter is provided on the lateral side 12 of the stem.

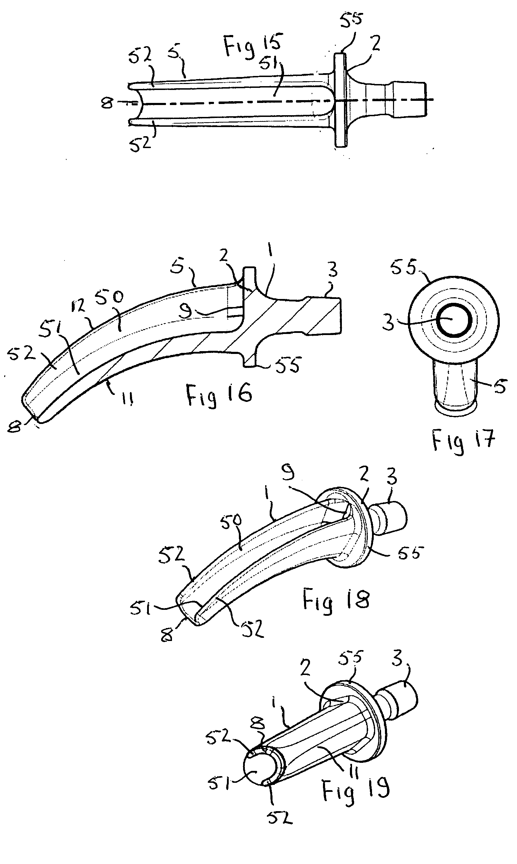

In the construction shown in Figures 11, 12, 13 and 14 the general configuration and shape is similar to that shown in Figures 7, 8, 9 and 10, inasmuch that the gutter and outer surface are both part-circular but in this arrangement the gutter 40 is provided on the medial side 11 of the stem 5. Again the wall thickness of the gutter decreases proximally to distally and the curve of the stem is similar to that described with regard to Figure 2. Figures 15, 16, 17, 18 and 19 show another construction which is similar in general shape to that described with regard to Figures 1, 2 and 3 and the same reference numerals are used to indicate similar parts but in this construction the gutter-shaped stem 5 has an outer surface which is part-circular and has a gutter 50 with a surface which has a lower semicircular portion 51 and extended upper side walls 52. With this type of construction the outer surface could alternative be part square or rectangular, as shown in Figures 1, 2 and 3.

The curve of the stem is similar to that shown in the other construction. As shown in Figures 15, 16, 17, 18 and 19 the gutter is on the lateral side 12 of the stem 5 but it could be alternatively provided on the medial side 11, as shown in the constructions illustrated in Figures 4 to 6 and 7 to 10. Figures 16, 17, 18 and 19 also show how a flange or collar 55 can be provided on the proximal end portion 2 of the stem if required for a particular type of surgery.

Collars similar to the collar 55 could be provided on any of the other constructions described above.

For a medial size femur of approximately 20 - 25 mm the length of stem 5 in the bone will be about 50 mm for a medium size stem.

The prosthesis can be made as a one-piece construction from any suitable metal material, for example titanium or stainless steel and shaped by, for example, moulding, casting, forging or any other process.

If desired the surface of the stem can be prepared to facilitate fixture, for example, by cement, or to assist bone growth.

## Claims

1. A short stem femoral prosthesis comprising a proximal end portion having or adapted to receive a bearing ball head, and a gutter-shaped stem having anterior and posterior sides which extends from said proximal end portion to an open distal end curved in a medial/lateral plane through its length and closed by a substantially radially extending proximal end wall adjacent said proximal end portion.

2. A short stem femoral prosthesis as claimed in claim 1 in which said gutter-shaped stem has a medial side which is formed as a curve of constant radius from a first centre point and a lateral side which is also formed as a constant radius curve from a second centre point so that the stem tapers from its proximal end to its distal end.

3. A short stem femoral prosthesis as claimed in claim 1 or claim 2 in which said gutter-shaped stem has an outer surface and an inner gutter surface which are both substantially square or rectangular in cross-section.

4. A short stem femoral prosthesis as claimed in claim 1 or claim 2 in which said gutter-shaped stem has an outer surface and an inner gutter surface which are both substantially square or rectangular in cross-section.

5. A short stem femoral prosthesis as claimed in claim 1 or claim 2 in which said gutter-shaped stem has an outer surface which is part-circular, square or rectangular and an inner gutter surface which has a semicircular lower portion and extended upper side walls in cross-section.

6. A short stem femoral prosthesis as claimed in any one of the preceding claims in which said gutter is provided in the medial side of the stem.

7. A short stem femoral prosthesis as claimed in any of preceding claims 1 to 5 in which said gutter is provided on the lateral side of the stem.

8. A short stem femoral prosthesis as claimed in any one of the preceding claims in which the thickness of the gutter is arranged to decrease proximally to distally.

9. A short stem femoral prosthesis as claimed in any one of the preceding claims in which the proximal end of the stem is provided with a projecting flange or collar.

10. A short stem femoral prosthesis as claimed in any one of the preceding claims in which the proximal end portion is provided with a tapered boss to receive a co-operating bearing ball.

11. A short stem femoral prosthesis as claimed in any one of the preceding claims which is made in one piece.

12. A short stem femoral prosthesis as claimed in any one of the preceding claims in combination with a bearing ball.
